# DEMANDE DE BREVET EUROPEEN

(11) **EP 0 589 809 A1**
(43) Date de publication de la demande: **30.03.1994**
(21) Numéro de dépôt: 93440071.4
(22) Date de dépôt: 01.09.1993
(51) Int. Cl.: A61F 2/16, A61L 27/00

(54) **Implant intra-oculaire de restauration ou correction spectrale**

(30) Priorité: 04.09.1992 FR 9210782
(71) Demandeur: LABORATOIRES DOMILENS S.A., F-69348 Lyon Cédex 07 (FR)
(72) Inventeur: Malbrel, Christian, F-51100 Reims (FR); Billard Etienne, F-39200 Saint-Claude (DE)

(57) **Abrégé**

Implant intra-oculaire, destiné à une insertion et mise en place dans l'oeil d'un patient, comprenant, d'une part une partie optique destinée à être traversée par la lumière incidente reçue par la cornée dudit oeil, constituée d'au moins un matériau transparent incorporant un adjuvant de transmission lumineuse, biocompatible avec les milieux internes dudit oeil, et d'autre part d'une partie haptique destinée à assurer le support de la partie optique dans l'oeil, caractérisé en ce que l'adjuvant de transmission lumineuse comprend au moins une substance filtrante, apte à modifier de manière contrôlée le spectre d'énergie de la lumière transmise par le matériau transparent, dans une bande sensible pour l'oeil dudit patient, commençant à une valeur au moins égale à 350 nm, en fonction d'une courbe de sensibilité lumineuse prédéterminée pour l'oeil dudit patient.

## Description

La présente invention concerne les implants intra-oculaires, susceptibles d'être insérés et mis en place dans l'oeil d'un patient, par voie chirurgicale, pour restaurer, modifier, ou corriger une ou plusieurs fonctions de l'oeil.

Aujourd'hui, tous les implants intra-oculaires connus et mis en oeuvre en chirurgie ophtalmique sont dédiés à la seule fonction optique de l'oeil humain, c'est-à-dire à la formation d'une image sur la rétine, au moyen de l'ensemble dioptrique constitué par la succession de la cornée, du cristallin, et du corps vitré. A cette fin, de manière générale, ces implants comprennent, d'une part, une partie optique en général circulaire, destinée à être traversée par la lumière incidente reçue par la cornée de l'oeil du patient, assurant la restauration (pour l'oeil aphaque) et/ou la correction (pour l'oeil phaque ou pseudo-phaque) des propriétés dioptriques du cristallin, et ayant à cet effet toute forme appropriée, par exemple convexe et/ou concave, et d'autre part une partie haptique, monobloc ou rapportée, destinée à assurer le support de la partie optique dans l'oeil. La partie optique peut être par ailleurs sphérique ou asphérique, à focale unique, ou multi-focale, à focale variable, etc...

Ces implants sont mis en place à tout endroit anatomiquement approprié de l'oeil, par rapport à la position du cristallin, à savoir dans la chambre antérieure, ou dans la chambre postérieure de l'oeil, notamment le sac capsulaire.

Dans le cas d'une implantation dans un oeil aphaque, il est connu de plus de protéger la rétine contre l'action néfaste des UV transmis par l'implant, et originellement présents dans tout éclairage naturel reçu par l'oeil, en incorporant dans le matériau transparent de la partie optique un adjuvant de transmission lumineuse, biocompatible avec les milieux internes de l'oeil, et absorbant au moins pour une large partie les UV du rayonnement solaire.

Par "adjuvant de transmission lumineuse", on entend tout composé ou mélange de composés, ajouté au matériau transparent de la partie optique, et susceptible de modifier les caractéristiques spectrales du rayonnement lumineux transmis par ladite partie optique en direction de la rétine de l'oeil.

Indépendamment de la fonction optique de l'oeil, différentes affections, maladies, anomalies, troubles, ou gênes, passagers ou permanents, de la vision résultent de l'inadéquation du spectre d'énergie de la lumière reçue par la rétine, par rapport à la courbe de sensibilité lumineuse de l'oeil, existante, souhaitable, ou désirée par l'ophtalmologiste.

Par "spectre d'énergie", on entend la répartition d'une énergie lumineuse en fonction des différentes longueurs d'ondes, visibles et invisibles, notamment ultra-violets (UV) et infra-rouges (IR).

Par "courbe de sensibilité", on entend la représentation de la sensibilité de l'oeil, en fonction des différentes longueurs d'ondes transmises à la rétine du patient. Cette courbe peut comprendre des longueurs d'ondes invisibles, telles qu'UV et IR, car on a mis en évidence ou on peut suputer aujourd'hui l'action réflexe de certaines longueurs d'ondes sur l'oeil, vis-à-vis de certaines fonctions organiques.

Ainsi, H.R Taylor, S. West, et B. Munoz (Department of Ophtalmology, Royal and Victorian Hospital, Melbourne), ont mis en évidence en 1992 que le ptérygion et la kératopathie en gouttelettes sont notamment associés à une exposition de la rétine à une couleur bleue intense (bande de longueurs d'ondes comprise entre 450 et 500 nm).

En conséquence, à des fins thérapeutiques, de soin, de prévention de certaines affections, ou de confort visuel, le besoin existe d'un moyen permettant une meilleure adaptation, voire une "synthonisation", du spectre d'énergie lumineuse reçu par la rétine de l'oeil, à la courbe de sensibilité réelle de ce dernier, ou à toute courbe de sensibilité "idéale", c'est-à-dire estimée utile par le praticien ophtalmologiste, pour restaurer, modifier ou corriger toute fonction de l'oeil autre que strictement d'optique géométrique, telle que perception des couleurs (par exemple trichromatie, dichromatie, monohromatie), des contrastes, etc...

Et tel est l'objet de la présente invention.

La présente invention concerne aux fins précitées un implant intra-oculaire, ayant éventuellement et additionnellement des caractéristiques de restauration et/ou correction optique, destiné à une insertion et mise en place dans l'oeil d'un patient. Cet implant comprend comme précédemment, d'une part une partie optique destinée à être traversée par la lumière incidente reçue par la cornée de l'oeil, constituée d'au moins un matériau transparent incorporant un adjuvant de transmission lumineuse, biocompatible avec les milieux internes dudit oeil, et d'autre part, une partie haptique destinée à assurer le support de la partie optique dans l'oeil. Mais à la différence des implants de pure correction optique, un implant selon l'invention assure une correction spectrale vis-à-vis de l'oeil du patient, et à cette fin, l'adjuvant de transmission lumineuse comprend au moins une substance filtrante, apte à modifier le spectre d'énergie de la lumière transmise par le matériau transparent de la partie optique, dans une bande sensible pour l'oeil dudit patient, commençant à une valeur au moins égale à 350 nm, et se terminant éventuellement à 800 nm, en fonction d'une courbe de sensibilité lumineuse prédéterminée pour l'oeil dudit patient, qu'il s'agisse de la courbe de sensibilité effective de sa rétine, ou d'une courbe de - sensibilité souhaitée ou idéale.

Par "substance filtrante", on entend toute substance ayant intrinsèquement l'aptitude de modifier d'une manière quelconque le spectre d'énergie de la lumière naturelle, dans une zone donnée de longueurs d'ondes, par exemple de diminuer l'énergie lumineuse dans ladite zone donnée. Il peut s'agir d'une substance colorée ou non, par exemple d'un colorant. Il peut s'agir d'une subtance absorbant différentes longueurs d'ondes, par exemple dans une zone comprise entre 400 et 620 nm. Il peut s'agir aussi de pigments, organiques ou minéraux, finement dispersés. La substance filtrante retenue doit être compatible avec le matériau transparent, ne pas affecter sa transparence, ni son homogénéité.

Toutes sortes de matériaux transparents peuvent être envisagés selon l'invention, qu'il s'agisse de matériaux traditionnels comme des verres spéciaux, ou de matériaux organiques tels que polymères, par exemple PMMA (polyméthylméthacrylate).

L'homme de métier dispose d'un éventail très large de substances filtrantes telles que définies précédemment, dont il connait les caractéristiques et propriétés spectrales, soit à partir des indications et prescriptions des fabricants, soit à partir de simples essais de routine. En conséquence, l'homme de métier est à même de sélectionner une ou plusieurs substances filtrantes, de les assembler, et en définitive de formuler un adjuvant de transmission lumineuse, tenant compte des propriétés spectrales intrinsèques du matériau transparent, pour aboutir à la modification contrôlée et souhaitée du spectre de la lumière transmise par l'implant. Par exemple, avec des colorants jaune (absorbant de 400 à 500 nm), orange (absorbant de 400 à 560 nm), et rouge (absorbant de 400 à 620 nm), l'homme de métier peut ajuster la composition spectrale de la lumière transmise dans les faibles longueurs d'onde.

Comme déjà dit, la formulation de l'adjuvant de transmission lumineuse dépend de la courbe de sensibilité lumineuse recherchée, laquelle constitue la réponse du patient aux signaux lumineux reçus par l'oeil. Comme on le sait, et en simplifiant beaucoup, cette réponse est le résultat, et de phénomènes purement physiques au niveau de la rétine, agissant comme un transducteur, et transformant les signaux lumineux en signaux électriques ou autres, et de phénomènes subjectifs d'interprétation de signaux électriques ou autres, reçus par le cortex cérébral en conséquence de la transduction.

En définitive, la courbe de sensibilité lumineuse intègre et globalise toutes les modifications de la répartition spectrale du rayonnement lumineux traversant l'oeil, depuis l'extérieur de la cornée jusqu 'à l'interception par la rétine. Et c'est pourquoi la formulation de l'adjuvant de transmission lumineuse tiendra compte des modifications spectrales apportées par d'autres éléments, telles que :
- l'association à l'implant de lunettes portées par le patient
- la présence ou non d'un autre implant dans l'oeil du patient, par exemple d'un implant de correction optique
- l'altération organique d'autres parties de l'oeil, par exemple de la cornée, provoquée par des causes naturelles (vieillissement), accidentelles, ou traumatiques (par exemple mise en place d'un implant).

A l'aide des instruments usuels d'examen de diagnostic de l'oeil, le praticien de l'art ophtalmique est à même de détecter, localiser, et éventuellement quantifier ces modifications spectrales préexistantes.

Un implant selon l'invention comporte ou non par ailleurs des caractéristiques géométriques de forme, lui conférant un rôle optique. Dans le premier cas, la partie optique de l'implant sera une lame du matériau transparent plus ou moins épaisse, par exemple une simple lame absorbante, et dans le second cas une lentille concave et/ou convexe.

Un implant selon l'invention est inséré et mis en place comme les implants aujourd'hui traditionnels, dans l'oeil aphaque, ou pseudo-phaque, ou phaque. Il peut donc être mis en place dans la chambre antérieure, ou dans la chambre postérieure, notamment dans le sac capsulaire.

La présente invention présente en outre les caractéristiques techniques suivantes :
- la substance filtrante est apte à modifier la lumière transmise par la partie optique dans au moins une zone dite de modulation, appartenant à la bande sensible pour l'oeil du patient ; par exemple, la substance filtrante est apte à diminuer l'énergie lumineuse transmise, dans cette zone de modulation ;
- comme déjà dit, l'adjuvant de transmission comprend plusieurs substances filtrantes, assemblées les unes aux autres pour obtenir la modulation contrôlée de la lumière transmise ;
- comme déjà dit, la partie optique présente une forme extérieure, notamment convexe et/ou concave, permettant de substituer au moins en partie le cristallin, ou de corriger l'optique de ce dernier ;
- l'adjuvant de transmission lumineuse représente entre 1 et 500 ppm du matériau transparent.

La présente invention est maintenant décrite par référence aux dessins annexés, dans lesquels :

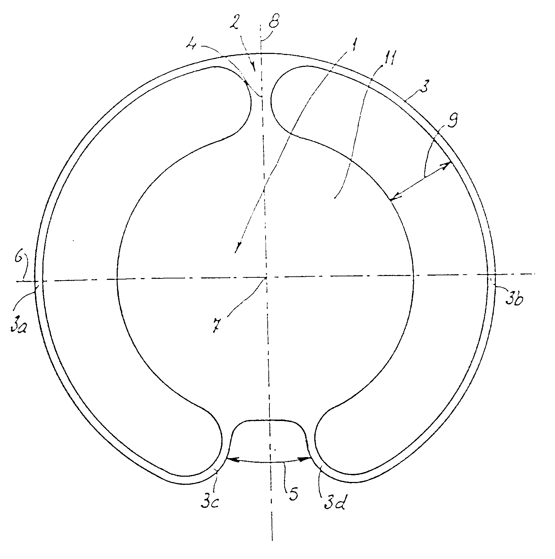

- les figures 1 et 2 représentent un implant conforme à la présente invention,
- la figure 3 représente l'oeil dans lequel est mis en place un implant selon l'invention.

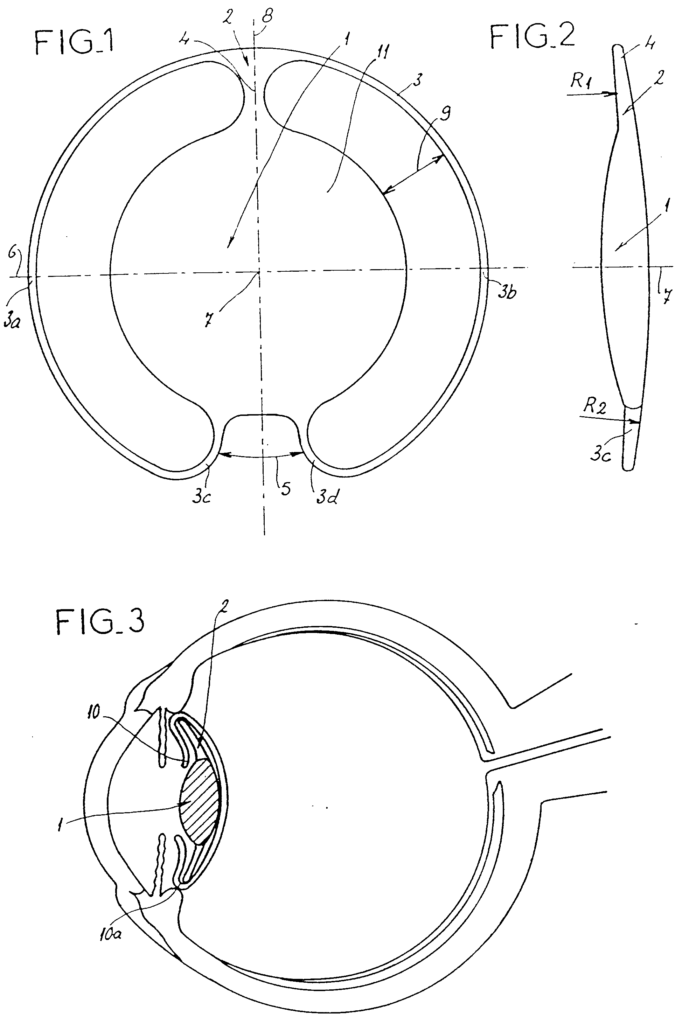

Un implant intra-oculaire 1 selon l'invention est destiné, comme représenté à la figure 3, à une insertion et mise en place dans l'oeil du patient, plus précisément dans le sac capsulaire 10 situé dans la chambre postérieure de l'oeil. Cet implant comprend, d'une part, une partie optique destinée à être traversée par la lumière incidente reçue par la cornée de l'oeil, constituée d'au moins un matériau transparent, par exemple un matériau plastique, modifié conformément à l'invention, et assurant par ailleurs une restauration des propriétés optiques du cristallin, et d'autre part une partie haptique 2, comportant deux anses 3a et 3b, destinées à assurer le support de la partie optique 11 dans l'oeil.

Conformément à l'invention, le matériau transparent utilisé pour la réalisation de l'implant monobloc, et par conséquent de sa partie optique, incorpore un adjuvant de transmission lumineuse, biocompatible avec les milieux internes de l'oeil, comprenant au moins une substance filtrante telle que définie précédemment, apte à modifier de manière contrôlée le spectre d'énergie de la lumière transmise par le matériau transparent, dans une bande sensible pour l'oeil du patient, commençant à une valeur au moins égale à 350 nm, et ceci en fonction d'une courbe de sensibilité lumineuse prédéterminée pour l'oeil dudit patient.

La présente invention s'appuie sur le protocole expérimental suivant.

Le patient opéré de la cataracte voit disparaître un filtre qui pendant de longues années a modifié sa perception de la lumière, des formes et des couleurs. Ce filtre naturel arrête tout particulièrement les radiations de courte longueur d'onde du spectre visible, entre 400 et 420 nm.

Son absence brutale crée chez l'aphaque ou chez le pseudo-phaque à implant clair, une impression d'éblouissement permanent, une chromatopsie à type de cyanopsie, et un trouble de la perception des contrastes, dès qu'il regarde une surface claire vivement éclairée. Si l'éblouissement est trop important, la chromatopsie devient une érythropsie, et le patient peut présenter un oedème maculaire transitoire ou définitif, faisant diminuer fortement son acuité visuelle centrale.

Pour pallier à ce phénomène, il a été introduit dans le matériau transparent de l'implant, un adjuvant filtrant arrêtant les ultra-violets en-dessous de 380 nm. Cette solution a diminué les phénomènes décrits ci-dessus sans les supprimer. En particulier, il suffit de laisser un patient opéré de la cataracte, pupille ouverte par de l'atropine, se déplacer à l'extérieur sans lunettes de protection pour observer l'apparition immédiate d'un oedème maculaire transitoire.

La suppression définitive de ce phénomène a été obtenue en introduisant des pigments dans le matériau transparent de la partie optique de l'implant, afin d'obtenir une coupure allant de 380 à 410 nm.

On a testé la vision- colorée, d'une part afin d'apprécier l'influence de l'implant modifié selon l'invention sur le sens chromatique du patient, et d'autre part afin d'obtenir des renseignements sur l'une des parties importantes de la vision.

Cette vision colorée a été testée à l'aide du test de 100 hue de Farnsworth, le patient étant placé à la lumière du jour toujours au même emplacement avec sa correction de près. Si ce test est précis et permet l'utilisation d'un score, il est par contre long d'utilisation, supérieur à trente minutes, étude du graphique comprise.

Ces tests ont mis en évidence une amélioration de la vision colorée du patient, avec un implant modifié selon l'invention.

On a également testé la sensibilité de la fovea en testant son seuil de détection d'un point de 10 minutes, présenté au sein de 4 points assurant la fixation. Ce seuil pour une fovea normale est supérieur ou égal à 26 db. Si l'oeil est protégé d'un trop grand éblouissement, ce seuil restera élevé, ce qui est le cas avec un implant selon la présente invention.

Enfin, on a étudié la sensibilité au contraste statique, en relevant les réponses obtenues lors de la présentation sur un écran cathodique d'un réseau horizontal composé de 6 fréquences spatiales allant de 0,8 à 26 cy/d°. On a démontré selon l'invention l'affaiblissement des réponses de basses fréquences spatiales, en cas d'oedème maculaire.

Les indications thérapeuthiques ou cliniques d'un implant intra-oculaire selon l'invention sont notamment les suivantes :
1°) Le traitement des phototraumatismes postérieurs à la mise en place d'un implant de restauration ou correction optique, induits notamment par les courtes longueurs d'onde du spectre visible ; la prévention du phénomène de chromatopsie immédiate, survenant après intervention de la cataracte, par excès d'illumination en teinte bleue ou violette.
2°) La prévention de l'oedème cystoïde du pseudophaque et de la dégénérescence maculaire liée à l'âge ; dans ce cas, l'adjuvant de transmission lumineuse est formulé pour diminuer l'illumination de la région maculaire par les courtes longueurs d'onde du spectre visible, ce qui évite la trop grande destruction du pigment visuel présent sur la macula, la formation trop importante de radicaux libres, et l'échauffement excessif de la région fovéale.
3°) L'amélioration de la perception centrale, par la filtration des courtes longueurs d'onde, ce qui évite d'exciter trop fortement les cônes bleus para-centraux, au détriment des cônes centraux sensibles au rouge et au vert.
4°) L'amélioration ou préservation de la perception des contrastes, en diminuant la sur-illumination dans les courtes longueurs d'onde des cellules fovéales, et donc la saturation des cellules ganglionnaires de type X.
5°) Le traitement de la rétinopathie diabétique de forme oedémateuse, en diminuant la quantité de lumière dans les courtes longueurs d'onde, arrivant au niveau du pôle postérieur, et l'échauffement excessif qui en résulte.
6°) Le traitement de l'albinisme, de la rétinite pigmentaire, de la degénérescence des cônes, par filtration des radiations toxiques de courte longueur d'onde du spectre visible.
7°) La stimulation de certaines fonctions de sécrétion réflexe de l'organisme, par exemple de l'hypophyse, par la transmission de certaines longueurs d'onde.

Dans les indications 1 à 5, la filtration nécessaire sera obtenue par l'incorporation d'un ou plusieurs pigments jaunes dans le matériau transparent, dans la proportion de 20 à 400 ppm, créant une correction spectrale entre 400 et 500 nm.

Dans les indications 6 et 7, la filtration nécessaire sera obtenue par l'incorporation d'un pigment orange dans le matériau transparent, et éventuellement dans de faibles proportions d'un pigment rouge en addition du pigment orange, ce qui permet d'absorber également des longueurs d'onde au-delà de 560 nm.

## Revendications

1. Matériau transparent incorporant un adjuvant de transmission lumineuse, biocompatible avec les milieux internes d'un oeil, caractérisé en ce que l'adjuvant de transmission lumineuse comprend au moins une substance filtrante, apte à modifier de manière contrôlée le spectre d'énergie de la lumière transmise par ledit matériau transparent, dans une bande dite sensible, commençant à une valeur au moins égale à 350 nm.

2. Matériau selon la revendication 1, caractérisé en ce que la substance filtrante est apte à modifier la lumière transmise, notamment diminuer l'énergie lumineuse transmise, dans au moins une zone de modulation, appartenant à la bande sensible.

3. Matériau selon la revendication 1, caractérisé en ce que l'adjuvant de transmission comprend plusieurs substances filtrantes, assemblées les unes aux autres pour obtenir la modulation de la lumière transmise.

4. Matériau selon la revendication 1, caractérisé en ce que l'adjuvant de transmission lumineuse représente entre 1 et 500 ppm du matériau transparent.

5. Implant intra-oculaire, destiné à une insertion et mise en place dans l'oeil d'un patient, comprenant, d'une part une partie optique destinée à être traversée par la lumière incidente reçue par la cornée dudit oeil, constituée d'au moins un matériau transparent selon l'une quelconque des revendications 1 à 4, et d'autre part d'une partie haptique destinée à assurer le support de la partie optique dans l'oeil, caractérisé en ce que l'adjuvant de transmission lumineuse est choisi en fonction d'une courbe de sensibilité lumineuse prédéterminée pour l'oeil dudit patient.

6. Implant selon la revendication 5, caractérisé en ce que la partie optique présente une forme extérieure, notamment convexe et/ou concave, permettant de substituer au moins en partie le cristallin, ou de corriger l'optique de ce dernier.
